Europäisches Patentamt

European Patent Office

Office européen des brevets

(19)

(11) Publication number: **0 202 667**
**A2**

## (12) EUROPEAN PATENT APPLICATION

(21) Application number: **86106889.8**

(22) Date of filing: **21.05.86**

(51) Int. Cl.⁴: **C 12 N 5/02**
**A 01 G 7/00**

(30) Priority: **21.05.85 JP 106887/85**

(43) Date of publication of application:
**26.11.86 Bulletin 86/48**

(84) Designated Contracting States:
**DE FR GB IT NL**

(71) Applicant: **MITSUI TOATSU CHEMICALS INC.**
**No. 2-5, Kasumigaseki 3-chome**
**Chiyoda-Ku Tokyo 100(JP)**

(72) Inventor: **Fujimura, Tatsuhito**
**Oizumigakuencho 8-12-18**
**Nerima-ku Tokyo(JP)**

(72) Inventor: **Sakurai, Motoi**
**2280-5, Inoguchi Nakai-cho**
**Ashigarakamigun Kanagawa(JP)**

(72) Inventor: **Akagi, Hiromori**
**Hase 4-1-28**
**Kamakura Kanagawa(JP)**

(72) Inventor: **Negishi, Tomoko**
**Honson 4-9-2-201**
**Chigasaki-shi Kanagawa(JP)**

(72) Inventor: **Hirose, Akira**
**7-3-14, Imaizumidai**
**Kamakura Kanagawa(JP)**

(74) Representative: **Kinzebach, Werner, Dr. et al,**
**Patentanwälte Reitstötter, Kinzebach und Partner**
**Sternwartstrasse 4 Postfach 86 06 49**
**D-8000 München 86(DE)**

(54) Method of regenerating rice plant from protoplasts.

(57) Method of generating a complete plant of rice from a protoplast thereof. To prepare the protoplast, a callus is derived from an embryo, scutellum, or radicle of a seed of rice. The callus is then cultured in a liquid medium to form a small cell cluster which readily releases a protoplast. The cell cluster is then treated with a protoplast-releasing enzymatic solution to obtain a protoplast. The protoplast is then cultured to form a callus, and the callus is then cultured in a differentiation medium to regenerate a whole plant of rice.

- 1 -

## BACKGROUND OF THE INVENTION

I. Field of the Invention

This invention relates to a method of forming a rice plant by using a so-called protoplast method. More specifically, this invention relates to a method of regenerating a rice plant from a callus derived from a protoplast which is derived from a callus originated from an embryo, scutellum or radicle of a seed of rice.

II.　Description of the Prior Art

Protoplast is a cell of a plant, bacterium, fungus and the like from which the cell wall has been removed. Since the protoplast does not have a cell wall, it is easily subjected to an artificial manipulation such as cell fusion, gene manipulation and artificial somatic cell mutation. Thus, if a complete plant can be regenerated from a protoplast manipulated, it would be possible to obtain a plant which has an advantageous characteristic which the wild type plant does not have. As a first step in developing this kind of so-called protoplast method, it is necessary to establish a technique for each of the particular organisms in interest, by which a complete plant can be regenerated from a protoplast.

Some techniques are known for dicotyledons such as tobacco by which a complete plant can be regenerated from a protoplast. The conventional techniques in this field include culturing methods of the protoplast by embedding the protoplast in a semi-solid agar medium, by suspending the protoplast in a liquid medium, and by culturing the protoplast using feeder cells. However, it has been found that these techniques are not effective for culturing a protoplast of rice, and if a protoplast of rice is cultured by one of these conventional methods, the protoplast dies or cannot grow.

As for the culturing technique of the protoplast of rice, it has been reported that a callus was derived from a protoplast obtained from a cell lacking its nitrate reductase (Wakasa et al., J. Plant Physiol. 117: pp.223-231, 1984), and that a shoot was generated from a callus derived from a protoplast obtained from a callus of a pollen (Ohno et al., Japanese Journal of Breeding 35: pp.54-55, 1985). However, it has not yet been reported that a complete plant of rice was regenerated from a protoplast of rice.

On the other hand, it has been reported by many researchers that complete plants were regenerated from cultured cells of rice (Nishi et al., Nature 219: pp.508-509, 1968). However, these techniques do not utilize the protoplast. Further, it has been found that obtaining a protoplast from the cells having a high differentiation ability used in these techniques is difficult, and to culture the protoplast is also difficult.

Thus, if a technique by which a complete plant of rice can be regenerated from a protoplast is established, the research of creating a variety of rice having an advantageous characteristic may be greatly advanced.

## SUMMARY OF THE INVENTION

Accordingly, the object of the present invention is to provide a method by which a complete plant of rice (plants belonging to genus Oryza such as Oryza sativa, Oryza glaberrima, Oryza perennis and so on) can be regenerated from a protoplast.

In the method of the present invention, a first callus is derived from an embryo, scutellum, or radicle of a seed of rice, and the first callus is then cultured in a liquid medium to form a cell cluster ready to release a protoplast. The cell cluster is then treated with a protoplast-releasing enzymatic solution to form a protoplast. The protoplast is then cultured to form a second callus, and a complete plant is regenerated from the second callus by culturing the second callus in a differentiation medium.

By the present invention, a complete plant of rice (plants belonging to genus Oryza such as Oryza sativa, Oryza glaberrima, Oryza perennis and so on) was regenerated from a protoplast for the first time.

DETAILED DESCRIPTION OF THE PREFERRED EMBODIMENTS

In the first step of the method of the present invention, a callus having a high differentiation ability is derived from an embryo, scutellum, or radicle of a seed of rice. This can be accomplished by culturing a cell from the embryo, scutellum, or radicle of a seed of rice in an agar medium of MS medium (Murashige and Skoog, Physiol. Plant. 15, pp.473-479, (1962)), B5 medium (Gamborg et al., Exp. Cell Res. 50, pp. 151-158, (1968)), or N6 medium (Chu et al., Scientia Scinica 18, pp. 659-663), R2 medium (Ohira et al., Plant Cell Physiol. 14, pp.1113-1121), each of which contains 1) 0.01 - 1% by weight of yeast extract, 0.01 - 1% by weight of malt extract, 0.01 - 1% by weight of casein hydrolysate, or 5 - 20% by weight of potato extract, 2) 0.1 - 10 mg/l of 2,4-dichlorophenoxy acetic acid (hereinafter referred to as 2,4-D) which is a phytohormone, and 3) 0 - 5 mg/l of benzyl adenine or 0 - 5 mg/l of kinetin. The culturing temperature may be about 20 to about 30°C, and preferably about 26°C. The culture may be conducted in the form of subculture, and the callus is obtained from the subculture. The subculturing may be conducted, for example, once every four weeks until the callus is obtained.

The thus obtained callus has a high differentiation ability. However, it is difficult to directly obtain a protoplast from the callus. Thus, in the present invention, a small cell cluster still having high differentiation ability, which readily forms a protoplast is first obtained from the callus, and a protoplast is obtained from the cell cluster.

Accordingly, the second step of the present invention is to form a cell cluster still retaining a high differentiation ability, which readily releases a protoplast, from the callus obtained in the above-mentioned first step. This step may be conducted by culturing the callus in a liquid medium of MS, N6 or R2 medium each of which contains 1) 0.1 - 1% by weight of yeast extract, 0.1 - 1% by weight of malt extract, 0.1 - 1% by weight of casein hydrolysate, or 5 - 20% by weight of potato extract, 2) 0.1 - 10 mg/l of 2,4-D, and 3) 0 - 5 mg/l of benzyl adenine or 0 - 5 mg/l of kinetin. The population density of the cells in the culture media is preferably 0.3 to 3% by weight. The culturing temperature may be about 20 to about 30°C. The culture may be conducted in the form of subculture, and the cell cluster is obtained from the subculture. The subculturing may be conducted, for example, once a week. The culture may be conducted in the form of rotation culture in which the container of the liquid medium is rotated horizontally at a revolution rate of, for example, 70 rpm. By this culture, a cell cluster of 1 to 3 mm diameter which still retains high differentiation ability may be obtained.

The third step of the present invention is to obtain a protoplast from the cell cluster obtained in the second step. This may be accomplished by treating the cell cluster with a protoplast-forming enzymatic solution. The solution may contain, for example, 0.1 to 10% by weight, preferably 1 to 5% by weight of cellulase, 0.1 to 5% by weight, preferably 0.5 to 2% by weight of macerating enzyme, 0 to 5% by weight, preferably 0.1 to 1% by weight of calcium chloride, and 0 to 5% by weight, preferably 0.1 to 1% by weight of potassium salt of dextran sulfate. The solution may further contain 3 to 15% by weight of mannitol as an osmoticum to adjust the osmosis. The pH of the solution may be, for example, 5.5. The cell cluster may be, for example, incubated with shaking in the enzymatic solution for 4 to 8 hours at a temperature of 20 to 30°C. By this treatment, the cell cluster may release a large number of protoplasts. The enzymatic solution containing the protoplasts is filterd to remove undigested cell clusters, and the protoplasts may be collected by centrifuging the filtrate at, for example, 50g.

The fourth step of the method of the present invention is to culture the thus obtained protoplast to generate a callus. It is known that the differentiation ability of a callus is sharply decreased when the callus is continuously subcultured. If the differentiation ability of the callus is low, a complete plant cannot be obtained from the callus. It is thus important in this step to maintain the high differentiation ability of the cell to be cultured. This task was accomplished according to the present invention by culturing the protoplast in a conditioned medium of N6, B5, or R2 medium each of which contains 1) 0.1 - 1% by weight of yeast extract, 0.1 - 1% by weight of malt extract, 0.1 - 1% by weight of casein hydrolysate, or 5 - 20% by weight of potato extract, 2) 0.1 - 10 mg/l of 2,4-D, and 3) 0 - 5 mg/l of benzyl adenine or 0 - 5 mg/l of kinetin. The filtrate of the culture medium used in the above-mentioned second step may be conveniently used as the conditioned medium. In this case, fresh medium may be supplemented to the filtrate. Further, it has been found by the present inventors that the division of the protoplast may be promoted by adjusting the osmosis of the medium by sucrose, instead by a conventional osmoticum such as mannitol or a mixture of mannitol and sucrose. Thus, the conditioned media may contain sucrose of, for example, 0.4 M. The pH of the culture medium is preferably adjusted to 5.2 or less by, for example, hydrochloric acid. It is also preferred that the conditioned medium be supplemented with 2,4-D to promote the proliferation ability of the protoplast. Further, it is preferred that the container of the culture medium be coated with a hydrophilic support such as semi-solid agar medium, alginic acid, gelatin and so on containing an osmoticum and the culture medium be placed on the hydrophilic support layer such that the thickness of the culture medium is 100 to 400 $\mu$m, and preferably 200 to 300 $\mu$m. By so doing, the cells are exposed to air or oxygen to a greater degree than in the case where the medium is placed in a container which is not coated with an agar. This is because that without the agar coating, the medium becomes spherical water drops due to its surface tension so that the contact area with air per a unit volume of the medium is decreased, while if the medium is placed on an agar layer, the surface tention of the medium is decreased by the agar layer so that a medium layer of 200 to 300 $\mu$m thickness having a greater contact area with air can be obtained. It has been found by the present inventors that this aerobic condition of the culture is important to the growth of the protoplast. The preferred thickness of the agar layer is 1 mm or less, and more preferably 200 $\mu$m or less. As the culture proceeds, the culture medium may be diluted by supplementing a fresh medium containing less concentration of osmoticum such as glucose. The culture may be conducted at a temperature of about 20°C to 30°C, and preferably about 27°C until a callus is obtained. The preferred population density of protoplasts is $10^4$ to $10^7$ protoplasts/ml, and more preferably $10^5$ to $5 \times 10^6$ protoplasts/ml.

In the fifth step of the method of the present invention, a complete plant of rice is regenerated from the callus obtained in the fourth step by culturing the callus in a differentiation medium. The differentiation medium used in this step may be MS medium containing 1) 0.1 - 1% by weight of yeast extract, 0.1 - 1% by weight of malt extract, 0.1 - 1% by weight of casein hydrolysate, or 5 - 20% by weight of potato extract, and 2) 0 - 10 mg/l of benzyl adenine or 0 - 10 mg/l of kinetin. The culturing temperature may be about 20 to about 30°C. It is preferred to culture the callus in a growth medium before culturing in the differentiation medium. The growth medium may be R2, N6, or MS medium each of which containing 1) 0.1 - 1% by weight of yeast extract, 0.1 - 1% by weight of malt extract, 0.1 - 1% by weight of casein hydrolysate, or 5 - 20% by weight of potato extract, 2) 0.1 - 10 mg/l of 2,4-D, and 3) 0 - 5 mg/l of benzyl adenine or 0 - 5 mg/l of kinetin. The culturing temperature may be about 20 to about 30°C.

This invention will be more readily understood by the following examples. It should be noted that the following examples are described for the illustration purpose only and the scope of the invention is by no means limited thereto.

EXAMPLE 1 - I

Derivation of Calli with High Differentiation Ability and Subculture Thereof

Seeds of rice (Oryza sativa cultivar, variety: Nihonbare) were immersed in 70% aqueous solution of ethanol for one minute, and then immersed in an aqueous solution of sodium hypochlorite (chlorine content of 5% by weight) for 15 minutes. The seeds were then washed with sterilized distilled water three times and then sowed on N6 agar medium containing 0.3% by weight of casein hydrolysate, 2 ppm of 2,4-D, and 1 ppm of benzyl adenine. After culturing at 26°C for three weeks, calli was formed from the scutella of the seeds. These calli were subcultured once every four weeks in the same conditions.

EXAMPLE 1 - II

Derivation of Calli with High Differentiation Ability and Subculture

Immature seeds of rice (Oryza sativa cultivar, variety: Sasanishiki) were immersed in 70% aqueous solution of ethanol for one minute to sterilize the outer surface thereof, and then washed with sterilized water. Immature embryos were removed from husked seeds and the embryos were planted on MS agar medium containing 0.3% by weight of yeast extract and 2 ppm of 2,4-D. Calli were generated after culturing at $26^\circ$C for three weeks. These calli were subcultured once every four weeks in the same conditions.

EXAMPLE 1 - III

Derivation of Calli with High Differentiation Ability and Subculture

Sterilized seeds of rice (Oryza sativa cultivar, (variety: Koshihikari) were planted on a medium consisting of agar. After one week, radicles grown were cut off and were planted on B5 medium containing 2 ppm of 2,4-D. Calli were generated after culturing at $26^\circ$C for three weeks. These calli were subcultured once every four weeks in the same conditions.

EXAMPLE 2

Culture of the Derived Callus in a Liquid Medium

The calli obtained from the subculture in the Examples 1-I, 1-II and 1-III were transferred to and subcultured in the liquid medium shown in Table 1. Thirty milliliters each of the liquid media was placed in a 100 ml flask, and the cells were cultured in the media while rotating the flask at 70 rpm at $26^\circ$C. Subculture was conducted once a week. The cell content in the media was about 0.3% by weight at the beginning of the culture and about 3% by weight at the end of the culture.

Table 1     Composition of Liquid Medium

| Basal Medium | Organic Additives | 2,4-D | Kinetin | BA |
|:---:|:---:|:---:|:---:|:---:|
| MS | CH 0.1 wt% | 2.0mg/l | 0mg/l | 0mg/l |
| N6 | YE 0.3 wt% | 2.0mg/l | 0mg/l | 1.0 mg/l |
| R2 | PE 10.0 wt% | 5.0mg/l | 2.0mg/l | 0mg/l |
| R2 | CH 0.2 wt% | 1.0mg/l | 1.0mg/l | 0mg/l |

CH: casein hydrolysate

YE: yeast extract

PE: potato extract

BA: benzyl adenine

EXAMPLE 3

Isolation of Protoplasts

The cells cultured for 7 days after the subculture were used as the starting material in this step. The protoplast-releasing enzymatic solution was an aqueous solution containing 4.0% by weight of Cellulase Onozuka RS (commercially available from Yakult Pharmaceutical), 1.0% by weight of Macerozyme R-10 (commercially available from Yakult Pharmaceutical), 0.5% by weight of calcium chloride, 0.5% by weight of potassium salt of dextran sulfate, and 0.4 M of mannitol. The cells were gently shaked in this solution for 6 hours at 27°C to obtain protoplasts. Then the enzymatic solution containing the protoplasts was filtered to remove the undigested cell clusters, and the filtrate was centrifuged at 50 g for 5 minutes to precipitate the protoplasts. The precipitated protoplasts were washed three times with 0.4 M aqueous solution of glucose and were cultured in the next step.

EXAMPLE 4

Formulation of the Culture Medium for the Protoplasts (Conditioned Medium)

The culture medium in the above Example 2 was filtered to remove the cells. To 10 ml of the filtrate of the culture, were added 50 µl of 100 ppm 2,4-D solution and 0.4M sucrose, and pH of the solution was adjusted with 0.1 N HCl to 4.5. To this medium, the same but fresh basal medium (e.g., R2 medium in case where R2 medium was used in Example 2) containing 0.4 M of sucrose was added in the amount of 1/4 volume of the former medium.

EXAMPLE 5

Culture of the Protoplasts

Two hundred microliters of the thus formulated conditioned medium was placed in a plastic Petri dish of 35 mm diameter, of which bottom surface was coated with a thin agar layer, and 30 $\mu$l of a suspension of the protoplasts containing $10^6$ protoplasts/ml was added thereto, and the mixture was spreaded uniformly. After sealing the Petri dish, culture was conducted in the dark at $27^{\circ}$C.

After about a week, the first divisions were observed, and small cell clusters of about 100 $\mu$m diameter were formed after about one month (see Table 2). At this point, 200 $\mu$l of the same fresh basal medium but containing reduced amount of sucrose of 3% by weight was added to the culture. After two weeks from this addition, the same operation was repeated. After 2 months from the initial culture of the protoplasts, a small callus of 1 mm diameter was formed.

Table2    Number of Small Cell Clusters per Petri Dish After
1 Week Culture

| Basal Medium | Organic Additives | 2,4-D | Kinetin | BA | NCC |
|---|---|---|---|---|---|
| N6 | CH 0.3 wt% | 2.0mg/l | | 1.0mg/l | 1500 |
| N6 | YE 0.3 wt% | 2.0mg/l | | | 1500 |
| B5 | ME 0.5 wt% | 2.0mg/l | | 1.0mg/l | 1200 |
| R2 | PE 10.0 wt% | 5.0mg/l | 2.0mg/l | | 500 |
| R2 | YE 0.2 wt% | 1.0mg/l | 1.0mg/l | | 1000 |
| MS | YE 0.3 wt% | 2.0mg/l | | | 300 |
| MS | | 2.0mg/l | | | 0 |
| N6 | | 2.0mg/l | | | 0 |
| B5 | | 2.0mg/l | | 1.0mg/l | 0 |
| R2 | | 5.0mg/l | 2.0mg/l | | 30 |

EXAMPLE 6

Growing of Calli

The calli derived from the protoplasts were transferred to a callus growth medium to grow the calli at 26°C for about 2 weeks. The composition of the growth media (agar) and the growth rate of the calli are shown in Table 3.

Table 3    Composition of Growth Media and Growth Rate

| Medium No. | BM | Organic Additives | 2,4-D | Kinetin | BA | GR* |
|---|---|---|---|---|---|---|
| 1 | N6 | CH 0.3 wt% | 2.0mg/1 | | 1.0mg/1 | ++ |
| 2 | N6 | YE 0.3 wt% | 2.0mg/1 | | | ++ |
| 3 | B5 | ME 0.5 wt% | 2.0mg/1 | | 1.0mg/1 | + |
| 4 | R2 | PE 10.0 wt% | 5.0mg/1 | 2.0mg/1 | | ± |
| 5 | R2 | YE 0.2 wt% | 1.0mg/1 | 1.0mg/1 | | ++ |
| 6 | MS | YE 0.3 wt% | 2.0mg/1 | | | +++ |
| 7 | MS | | 2.0mg/1 | | | ++ |
| 8 | N6 | | 2.0mg/1 | | | ++ |
| 9 | B5 | | 2.0mg/1 | | 1.0mg/1 | + |
| 10 | R2 | | 5.0mg/1 | | 2.0mg/1 | + |

BM: Basal Medium

GR: Growth Rate

*   +++: Very High

++: High

 +: Medium

 ±: Scarcely Grows

EXAMPLE 7

Regeneration of Whole Plants

The calli grown on the growth media were differentiated to whole plants on the differentiation media (agar) shown in Table 4 at 26°C. One week after the transplantation, green spots were formed, and one month after the transplantation, small whole plants were regenerated.  The small whole plants were transplanted to an agar contanined in a large test tube, and whole plants of medium size were obtained.

Table 4    Composition of Differentiation Media and
Regeneration of Whole Plants

| | | Differentiation Medium | | | | |
|---|---|---|---|---|---|---|
| | BM | Organic Additives | Kinetin | BA | GM* | Redifferentiation** |
| | MS | CH   0.3 wt% | 5mg/l | | 1 | Good |
| | MS | CH 0.3 wt% | 5 mg/l | | 8 | Not Observed |
| | MS | YE 0.3 wt% | 5 mg/l | | 1 | Observed |
| | MS | ME 0.3 wt% | 5 mg/l | | 1 | Observed |
| | MS | YE 0.3 wt% | | 5mg/l | 2 | Observed |
| | MS | YE 0.3 wt% | | 5mg/l | 8 | Not Observed |
| | MS | ME 0.5 wt% | 5 mg/l | | 3 | Observed |
| | MS | ME 0.5 wt% | 5 mg/l | | 9 | Not Observed |
| | MS | PE 10.0 wt% | 5 mg/l | | 6 | Observed |
| | MS | PE 10.0 wt% | 5 mg/l | | 7 | Not Observed |
| | MS | | 5 mg/l | | 1 | Not Observed |
| | MS | | 5 mg/l | | 8 | Not Observed |
| | MS | | | 5mg/l | 1 | Not Observed |
| | MS | | 5 mg/l | 8mg/l | 8 | Not Observed |
| | MS | | 5 mg/l | | 3 | Not Observed |
| | MS | | 5 mg/l | | 9 | Not Observed |
| | N6 | CH 0.3 wt% | 5 mg/l | | 1 | Not Observed |
| | B5 | YE 0.3 wt% | 5 mg/l | | 1 | Not Observed |
| | R2 | ME 0.5 wt% | 5 mg/l | | 1 | Not Observed |

*GM: Growth Medium (the numbers in the column shows the
medium No. in Table 3)

**Redifferentiation: Redifferentiation to a Whole Plant

Claims

1. A method of forming a rice plant comprising, in the order mentioned, the steps of:

deriving a first callus from an embryo, scutellum, or radicle of a seed of rice;

culturing the first callus in a liquid medium to form a cell cluster which readily releases a protoplast;

treating the cell cluster with a protoplast-releasing enzymatic solution to release a protoplast;

culturing the protoplast to form a second callus; and

regenerating a rice plant from the second callus.

2. The method of claim 1 wherein the first step is conducted by culturing the embryo, scutellum or radicle in MS, B5, N6, or R2 agar medium each of which containing 1) 0.01 - 1% by weight of yeast extract, 0.01 - 1% by weight of malt extract, 0.01 - 1% by weight of casein hydrolysate, or 5 - 20% by weight of potato extract, 2) 0.1 - 10 mg/l of 2,4-D, and 3) 0 - 5 mg/l of benzyl adenine or 0 - 5 mg/l of kinetin.

3. The method of claim 2 wherein culturing the embryo, scutellum or radicle is conducted in the form of a subculture.

4. The method of claim 1 wherein the second step is conducted by culturing the first callus in R2, N6 or MS medium each of which containing 1) 0.1 - 1% by weight of yeast extract, 0.1 - 1% by weight of malt extract, 0.1 - 1% by weight of casein hydrolysate, or 5 - 20% by weight of potato extract, 2) 0.1 - 10 mg/l of 2,4-D, and 3) 0 - 5 mg/l of benzyl adenine or 0 - 5 mg/l of kinetin, the cell concentration in the medium being 0.3 - 3% by weight.

5. The method of claim 4 wherein culturing the first callus is conducted in the form of a subculture.

6. The method of claim 1 wherein the protoplast-releasing enzymatic solution is an aqueous solution contaning 0.1 to 10% by weight of cellulase, 0.1 to 5% by weight of macerating enzyme, 0 to 5% by weight of calcium chloride, 0 to 5% by weight of potassium salt of dextran sulfate, and 3 to 15% by weight of mannitol.

7. The method of claim 1 wherein the fourth step is conducted by culturing the protoplast in a conditioned medium derived from the medium used in the step 2, the conditioned medium being placed on a hydrophilic support layer containing an osmoticum, and the thickness of the conditioned medium placed on the hydrophilic support layer being 200 to 300 µm.

8. The method of claim 7 wherein the osmosis of the conditioned medium is adjusted by sucrose, and the pH of the conditioned medium is adjusted to not more than 5.2.

9. The method of claim 7 wherein the hydrophilic support is made of agar, alginic acid or gelatin.

10. The method of claim 1 wherein the fifth step is conducted by culturing the second callus in a differentiation medium which is the MS medium contaning 1) 0.1 - 1% by weight of yeast extract, 0.1 - 1% by weight of malt extract, 0.1 - 1% by weight of casein hydrolysate, or 5 - 20% by weight of potato extract, and 2) 0 - 5 mg/l of benzyl adenine or 0 - 5 mg/l of kinetin.

11. The method of claim 1 wherein the rice is Oryza sativa.

12. The method of claim 10 wherein the fifth step further comprises the step of growing the second callus in MS, B5, N6, or R2 medium each of which containing 1) 0.1 - 1% by weight of yeast extract, 0.1 - 1% by weight of malt extract, 0.1 - 1% by weight of casein hydrolysate, or 5 - 20% by weight of potato extract, 2) 0.1 - 10 mg/l of 2,4-D, and 3) 0 - 5 mg/l of benzyl adenine or 0 - 5 mg/l of kinetin, before culturing in a differentiation medium.

13. A method of forming a rice plant comprising, in the order mentioned, the steps of:

culturing an embryo, scutellum, or radicle of a seed of rice in MS, B5, N6, or R2 agar medium each of which containing 1) 0.01 - 1% by weight of yeast extract, 0.01 - 1% by weight of malt extract, 0.01 - 1% by weight of casein hydrolysate, or 5 - 20% by weight of potato extract, 2) 0.1 - 10 mg/l of 2,4-D, and 3) 0 - 5 mg/l of benzyl adenine or 0 - 5 mg/l of kinetin to form a first callus;

culturing the first callus in R2, N6 or MS liquid medium each of which containing 1) 0.1 - 1% by weight of yeast extract, 0.1 - 1% by weight of malt extract, 0.1 - 1% by weight of casein hydrolysate, or 5 - 20% by weight of potato extract, 2) 0.1 - 10 mg/l of 2,4-D, and 3) 0 - 5 mg/l of benzyl adenine or 0 - 5 mg/l of kinetin to form a cell cluster which readily releases a protoplast, the cell concentration in the medium being 0.3 - 3% by weight;

treating the cell cluster with a protoplast-releasing enzymatic aqueous solution containing 0.1 to 10% by weight of cellulase, 0.1 to 5% by weight of macerating enzyme, 0 to 5% by weight of calcium chloride, 0 to 5% by weight of potassium salt of dextran sulfate, and 3 to 15% by weight of mannitol to release a protoplast of rice;

culturing the protoplast in a conditioned medium derived from the medium used in the step 2 to form a second callus, the conditioned medium being placed on a hydrophilic support layer, and the thickness of the conditioned medium placed on the agar layer being 200 to 300 μm; and

culturing the second callus in a differentiation medium which is the MS medium contaning 1) 0.1 - 1% by weight of yeast extract, 0.1 - 1% by weight of malt extract, 0.1 - 1% by weight of casein hydrolysate, or 5 - 20% by weight of potato extract, and 2) 0 - 5 mg/l of benzyl adenine or 0 - 5 mg/l of kinetin to form a rice plant.

14. The method of claim 12 wherein the rice is Oryza sativa.